# FASCICULE DE BREVET EUROPEEN

(11) **EP 3 638 062 B1**
(45) Date de publication et mention de la délivrance du brevet: **15.05.2024**
(21) Numéro de dépôt: 18737007.7
(22) Date de dépôt: 15.06.2018
(51) Int. Cl.: A41D 13/00, A61F 5/02, A41D 31/18, A41B 9/06, A61F 5/01

(54) **ARTICLE VESTIMENTAIRE D'ASSISTANCE BIOMECANIQUE**
BEKLEIDUNGSSTÜCK MIT BIOMECHANISCHER UNTERSTÜTZUNG
ARTICLE OF CLOTHING WITH BIOMECHANICAL ASSISTANCE

(30) Priorité: 16.06.2017 FR 1755468
(43) Date de publication de la demande: 22.04.2020
(73) Titulaire: Barre, Bertrand, 01330 Lapeyrouse (FR)
(72) Inventeur: Barre, Bertrand, 01330 Lapeyrouse (FR)
(74) Mandataire: Talbot, Alexandre
(86) Numéro de dépôt international: PCT/FR2018/051435
(87) Numéro de publication internationale: WO 2018/229451

(56) Documents cités:
- EP-A1- 2 923 678
- WO-A1-2016/185029
- GB-A- 2 527 629
- JP-A- 2006 320 640
- US-A1- 2013 012 853

## Description

La présente invention concerne un article vestimentaire ou vêtement destiné, agencé, ou adapté pour exercer une assistance biomécanique à une partie du corps de l'utilisateur ou de l'utilisatrice le portant, c'est-à-dire lorsque ledit article vestimentaire revêt cet utilisateur.

Par assistance biomécanique, on entend que l'article vestimentaire, porté par le corps de l'utilisateur, exerce une action de type mécanique sur ce corps, que ce dernier soit statique ou en mouvement, et que cette action s'exerce sur le squelette, les muscles, les ligaments, les tendons, ou les articulations du corps revêtu par l'article vestimentaire.

Cette action biomécanique a différentes finalités, notamment qu'il s'agisse de soutenir, protéger, modifier ou corriger la position du corps de l'utilisateur revêtu par l'article vestimentaire.

Plus spécifiquement, la présente invention concerne un article vestimentaire d'assistance biomécanique à tout ou partie du tronc de l'utilisateur ayant revêtu ledit article.

Le tronc s'entend de l'ensemble du corps humain qui se trouve entre la ceinture scapulaire (ligne des épaules) et la ceinture pelvienne (ligne du bassin). Le tronc comprend le torse qui s'entend de l'ensemble constitué par les épaules, le thorax, et la partie supérieure de l'abdomen, dont le bassin. Et le tronc, qui s'étend de la base de la nuque au bas du dos, englobe ou correspond à la colonne vertébrale, ou rachis, constitué de bas en haut, par cinq vertèbres lombaires L1 à L5, se terminant par le sacrum et le coccyx, douze vertèbres dorsales D1 à D12, et sept vertèbres cervicales C1 à C7.

La présente invention a pour objet un article vestimentaire d'assistance biomécanique au tronc de l'utilisateur revêtu avec ledit article, permettant d'activer ou stimuler plus particulièrement tout ou partie de la colonne vertébrale dudit utilisateur.

Cet article vestimentaire comprend ou incorpore une gaine élastique d'assistance fonctionnelle à au moins un segment longitudinal d'intérêt du tronc de l'utilisateur revêtu par ledit article. A titre indicatif, mais non limitatif, cette gaine présente donc une certaine hauteur, parallèlement à l'axe longitudinal de l'article vestimentaire, pour recouvrir ou appuyer, dans sa partie postérieure, comme défini ci-après, sur au moins trois vertèbres consécutives, matérialisant en hauteur le segment longitudinal d'intérêt du tronc de l'utilisateur revêtu par ladite gaine.

Conformément au document US 2013/0012853, est décrit un article vestimentaire ou vêtement selon le préambule de la revendication 1, du type maillot de corps sans manches ou brassière d'assistance biomécanique au torse de l'utilisateur, c'est-à-dire de la personne ayant revêtu ou portant ledit article vestimentaire ou vêtement.

Conformément au document WO 2016/185 029, est décrit un article vestimentaire ou vêtement de correction de la position des épaules de l'utilisateur, c'est-à-dire de la personne ayant revêtu ou portant ledit article. Cet article comprend une gaine élastique d'assistance fonctionnelle. Deux séries de sangles, de rappel, disposées obliquement de part et d'autre de l'ouverture pour le cou de l'utilisateur, chevauchent les deux épaules de ce dernier portant le vêtement. Chaque sangle s'étend librement au-dessus de la gaine élastique, entre deux extrémités coulissant librement respectivement sur un barreau antérieur et un barreau postérieur, rapportés sur ladite gaine élastique. Les sangles sont ainsi réglables en position en fonction de la morphologie de l'utilisateur, et de la correction posturale à apporter.

Conformément au document GB 2 527 629, est décrit un article ou vêtement de correction posturale comportant une gaine élastique d'assistance fonctionnelle. Deux bandes obliques de rappel sont cousues l'une à l'autre, à une extrémité dans la partie postérieure et supérieure du vêtement. Ces deux bandes s'étendent à ou selon la surface de la gaine, à partir de la couture postérieure centrale de jonction vers la partie antérieure de la gaine, en chevauchant respectivement les deux épaules de l'utilisateur, jusqu'à leurs extrémités opposées ménageant sur la face antérieure du vêtement une portion centrale libre de tout ceinturage, en dehors de celui procuré par le port du vêtement, dont la gaine élastique.

Conformément au document EP 2 923 678, est décrit un article vestimentaire d'assistance biomécanique au torse de l'utilisateur, plus particulièrement de correction posturale, sur lequel est rapportée une structure totalement élastique monolithe ouverte, constituée par, premièrement un élément longitudinal central postérieur, deuxièmement une ceinture inférieure, liée postérieurement et perpendiculairement à l'élément longitudinal central postérieur, et troisièmement deux sangles, chevauchant respectivement les deux épaules de l'utilisateur, liées chacune à une extrémité postérieure et à l'extrémité opposée à la ceinture.

Conformément au document JP 2006 320 640, est décrit un article vestimentaire d'assistance biomécanique du même type que celui décrit par le document EP 2 923 678.

Toutes les solutions d'assistance biomécanique au torse de l'utilisateur, décrites précédemment, ont des constructions textiles relativement hétérogènes, au sens où les différents éléments constitutifs de l'article vestimentaire intégrés à ou rapportés dans la gaine élastique, demeurent relativement indépendants les uns des autres. Consécutivement, un tel vêtement peut certes apporter une assistance fonctionnelle locale, voire ponctuelle, au torse de l'utilisateur, mais à l'exclusion de toute action biomécanique plus globale ou coordonnée de la gaine vis-à-vis dudit torse.

La présente invention a pour objet un article vestimentaire, ou vêtement, dont la gaine élastique se trouve renforcée par un agencement textile structurant dans l'espace, notamment selon trois directions orthogonales, l'assistance fonctionnelle ou biomécanique qu'elle apporte au torse de l'utilisateur.

Conformément à la présente invention, un article vestimentaire selon la revendication 1 est proposé. Des modes de réalisation préférentiels de l'invention sont proposés dans les revendications dépendantes.

L'élément longitudinal postérieur central, relativement rigide transversalement, disposé dans ou sur la gaine élastique de l'article vestimentaire d'assistance biomécanique selon la présente invention, se comporte mécaniquement comme un barreau postérieur d'ancrage transversal de la gaine élastique, au sens où à la jonction de cette dernière avec ledit élément longitudinal postérieur central, ce dernier n'a pratiquement pas ou peu d'élasticité transversale.

A raison de l'existence de ce barreau postérieur d'ancrage transversal, l'élasticité périmétrique de la gaine élastique se trouve répartie de part et d'autre de ce dernier, autour du segment longitudinal d'intérêt du tronc de l'utilisateur, par exemple son torse.

La compression complète exercée en rappel sur ce segment longitudinal d'intérêt est relativement importante, du fait du taux d'élasticité significatif de la gaine, obtenu par construction textile de cette dernière, en particulier par le choix des fils élastiques tissés et/ou tricotés pour l'obtenir. Ce taux d'élasticité important se matérialise par le fait que, en considérant l'article vestimentaire, à l'état initial, c'est-à-dire non porté ou revêtu par l'utilisateur, et à l'état déployé, celui-ci présente une petite taille au regard du segment longitudinal d'intérêt du tronc de l'utilisateur, requérant un effort important de ce dernier pour le revêtir et ainsi l'élargir à l'encontre du rappel centripète élastique important de la gaine.

La gaine élastique selon la présente invention apporte une assistance biomécanique au torse de l'utilisateur, plus précisément une force de rappel élastique de la partie antérieure de l'article vestimentaire, ou vêtement, vers la partie postérieure de ce dernier. Cette force de rappel élastique se trouve distribuée ou répartie en surface à partir du barreau postérieur d'ancrage transversal constitué par l'élément longitudinal postérieur central, de manière symétrique par rapport au plan sagittal du vêtement, de sa partie postérieure vers sa partie antérieure, grâce aux deux sections textiles de rappel élastique rayonnant chacune à partir du barreau postérieur d'ancrage transversal, lui étant liées par construction textile, le cas échéant, et pouvant aboutir du côté antérieur du vêtement selon une bordure continue, de profil divers ou varié.

Le terme "section textile" réfère, selon sa signification usuelle, à une pièce ou un élément surfacique délimité par une bordure, ayant une extension continue ou discontinue (ajouré par exemple) en surface, et susceptible de ne présenter, considéré individuellement, aucune symétrie particulière ou dimension spécifique (par exemple sa longueur par rapport à sa largeur).

Une section textile, telle que considérée par la présente invention, peut donc prendre les formes les plus diverses et variées, par exemple nappe ou voile textile élastique, intégré ou compris dans l'épaisseur de la gaine élastique d'assistance fonctionnelle, ou fixé à l'intérieur et/ou à l'extérieur de ladite gaine. Dans certains cas, une section textile considérée par la présente invention n'est pas une bande textile.

Au total, les caractéristiques textiles définies et discutées précédemment permettent d'intégrer dans un vêtement selon la présente invention une exo-structure de contrôle selon ses trois axes anatomiques (antéro-postérieur, transversal, et longitudinal) définis ci-après, de l'assistance biomécanique apportée à l'utilisateur, et en particulier du rappel élastique postural de son torse.

La présente invention, à la différence du document US2013/0012853, retient l'élasticité propre, ou les élasticités propres selon différentes directions de cette exo-structure, rapportée dans ou sur la gaine élastique, et constituée par l'articulation des deux sections textiles de rappel sur l'élément longitudinal postérieur central d'ancrage transversal, comme paramètre(s) déterminant ou contrôlant l'assistance biomécanique au torse de l'utilisateur.

Et, selon l'invention, cette élasticité propre doit être différenciée, d'abord par rapport celle de la gaine élastique; et, ensuite, un même constituant de cette exo-structure, par exemple l'élément longitudinal postérieur central, doit avoir des élasticités différenciées selon les directions de référence considérées.

Selon l'invention, c'est en coordonnant et contrôlant ces différentes élasticités dans un même article vestimentaire que l'on apporte une assistance biomécanique au torse de l'utilisateur.

En d'autres termes, selon la présente invention, la gaine élastique, par sa construction textile définie ci-après, présente en combinaison les caractéristiques techniques suivantes :
a) de part et d'autre de l'élément longitudinal postérieur central d'ancrage transversal défini ci-après, cette gaine élastique présente une élasticité périmétrique, autour de l'axe longitudinal de l'article, et selon au moins une partie de sa hauteur, déterminant à l'état déployé un volume interne, initial, c'est-à-dire lorsque l'article vestimentaire n'est pas porté par l'utilisateur, au plus égal à 90 % du volume externe du segment longitudinal d'intérêt du tronc de l'utilisateur ; à titre indicatif, mais non limitatif, ce volume externe est sensiblement égal au volume interne sous tension, final, de la gaine élastique, lorsque l'utilisateur a revêtu l'article vestimentaire, la gaine ceignant alors le segment longitudinal d'intérêt de son tronc ;
b) cette gaine comporte au moins un élément longitudinal postérieur central d'ancrage transversal, dont l'élasticité transversale est plus faible que son élasticité longitudinale ; cet élément longitudinal postérieur central s'aligne avec ou sur la colonne vertébrale de l'utilisateur ayant revêtu l'article vestimentaire ; à titre indicatif, mais non limitatif, cet élément longitudinal définit, par tous moyens de réalisation ou obtention appropriés, sur la gaine élastique, au moins une bande longitudinale postérieure, rigide au sens où celle-ci n'a pratiquement aucune élasticité transversale, c'est-à-dire selon sa largeur, tout en demeurant déformable avec le reste de la gaine élastique ;
c) de part et d'autre de l'élément longitudinal postérieur central d'ancrage transversal, la gaine élastique comporte deux sections textiles de rappel élastique du tronc, et notamment du torse de l'utilisateur revêtu par l'article vestimentaire ; chaque section de rappel élastique s'étend à ou selon la surface de la gaine élastique, à partir dudit élément longitudinal postérieur, vers la partie antérieure de la gaine, jusqu'à une bordure continue dont une ou plusieurs portions sont situées en dehors ou au-delà du côté postérieur de la gaine élastique ; chaque section textile de rappel élastique présente une élasticité transversale, c'est-à-dire, à titre indicatif, mais non limitatif, selon la largeur de ladite section, moins importante que l'élasticité transversale, c'est-à-dire à titre indicatif, mais non limitatif, que l'élasticité périmétrique ou transversale de la gaine élastique ; et ces deux sections textiles de rappel élastique laissant au moins une portion centrale et antérieure de ladite gaine, libre de tout ceinturage, outre celui élastique inhérent au port de l'article vestimentaire, et donc de la gaine élastique d'assistance fonctionnelle.

Les termes "longitudinal", "transversal", "postérieur", "antérieur", utilisés précédemment sont définis dans un repère selon lequel l'utilisateur portant ou ayant revêtu l'article vestimentaire selon la présente invention est dans la position standard anatomique humaine, selon laquelle l'utilisateur ainsi revêtu est en position debout face à l'observateur.

Dans cette position, on définit trois plans imaginaires passant par le centre de gravité du corps de l'utilisateur, et qui sont perpendiculaires les uns par rapport aux autres, à savoir :
- le plan sagittal, vertical, qui passe par la ligne médiane du corps ou du torse de l'utilisateur, et qui le divise en deux parties symétriques, droite et gauche ;
- le plan frontal, vertical, perpendiculaire au plan sagittal, qui divise le torse de l'utilisateur, en deux parties symétriques, antérieure et postérieure (dorsale) ;
- et le plan transversal, horizontal, parallèle au sol, qui divise le corps de l'utilisateur en deux parties symétriques, supérieure du côté du tronc, et inférieure.

L'intersection de ces trois plans définit trois axes anatomiques, par rapport auxquels l'article vestimentaire selon l'invention, porté par l'utilisateur, peut être défini ou décrit, à savoir :
- l'axe antéro-postérieur formé par l'intersection des plans sagittal et transversal, perpendiculaire au plan frontal ;
- l'axe transversal formé par l'intersection des plans frontal et transversal, perpendiculaire au plan sagittal ;
- l'axe longitudinal formé par l'intersection des plans sagittal et frontal, perpendiculaire au plan transversal.

Un article vestimentaire selon la présente invention, lorsqu'il est porté par l'utilisateur, conduit à une déformation élastique centrifuge vers l'extérieur de la gaine élastique d'assistance fonctionnelle, autour de l'axe longitudinal de l'article vestimentaire, la gaine étant rappelée élastiquement de manière centripète vers l'intérieur, en comprimant le tronc, et plus précisément le segment longitudinal d'intérêt du tronc de l'utilisateur.

Dans cette conformation déformée élastiquement de la gaine, alors assimilable à une "seconde peau" du tronc de l'utilisateur, l'élément longitudinal postérieur central d'ancrage transversal vient et demeure en appui relativement ferme ou rigide, et en position verticale, contre au moins les trois vertèbres consécutives couvertes par la gaine élastique d'assistance fonctionnelle.

Dès lors, toujours dans cette conformation déformée, les déformations supplémentaires de la gaine élastique d'assistance fonctionnelle s'effectuent de part et d'autre de l'élément longitudinal postérieur central d'ancrage transversal, c'est-à-dire, au porté de l'article vestimentaire par l'utilisateur, de part et d'autre de la colonne vertébrale de l'utilisateur.

Ces déformations supplémentaires sont sous le contrôle, symétriquement par rapport à la colonne vertébrale de l'utilisateur, exercé par le rappel des deux sections textiles de rappel élastique respectivement, ancrées et articulées chacune sur l'élément longitudinal postérieur central, et travaillant à la surface de la gaine élastique, en s'ouvrant et/ou en se refermant, mais jamais en ceinturant de façon concentrique le segment longitudinal d'intérêt du tronc de l'utilisateur, en particulier sans comprimer son sternum.

Au total, au porté, un article vestimentaire selon l'invention, quoique comprimant élastiquement le tronc de l'utilisateur, exerce une action biomécanique sur celui-ci, effective et ressentie, mais sans ceinturage, réel et oppressant, du tronc, dont le torse du même utilisateur.

Par "élasticité" et/ou "élastique", selon la présente invention, on entend la capacité d'un élément de surface, considéré à plat ou en volume, d'épaisseur relativement faible, ou de tout élément allongé tel qu'une mèche ou un fil, à s'allonger linéairement sous une contrainte mécanique longitudinale ou curviligne, en tension, tout en reprenant sa conformation d'origine lorsque la contrainte disparaît.

Cette élasticité peut être mesurée, avant rupture, selon différentes méthodes et avec différents appareils, bien connus de l'homme du métier, en particulier dans l'industrie textile. Certaines de ces méthodes sont d'ailleurs normalisées.

En général, sous contrainte mécanique raisonnable, l'allongement de l'élément de surface, ou de l'élément allongé, sous observation ou mesure, est proportionnel à la force d'étirement, selon le module de Young, exprimé en Pa.

Plus simplement, dans l'industrie textile, et en particulier celle de maille ou du tricot, l'élasticité peut être évaluée en mesurant le pourcentage d'allongement de la surface ou d'une bande textile sous observation ou mesure, sous une contrainte d'allongement prédéterminée. Un tissu faiblement élastique aura un pourcentage d'allongement plus faible que celui d'un tissu fortement élastique ; de même pour un fil ou bande textile faiblement élastique, par rapport à un fil ou bande fortement élastique.

La présente invention est maintenant décrite à titre d'exemple par référence aux dessins annexés, représentant schématiquement un maillot ou T-shirt à manches courtes M d'assistance biomécanique au tronc, notamment au torse de l'utilisateur U, en ce compris sa ceinture occipitale et/ou sa ceinture scapulaire, et au moins les trois vertèbres consécutives qui leur correspondent, dessins selon lesquels :
- les figures 1 et 2 représentent le maillot M, porté par l'utilisateur U, respectivement vu de devant et vu de dos ;
- la figure 3 représente ce même maillot M, vu de face, à l'état initial, c'est-à-dire lorsque le maillot n'est pas porté par l'utilisateur U, mais à l'état déployé ;
- la figure 4 représente le même maillot M, dans la même conformation, vu en coupe IV-IV de la figure 3 ;
- la figure 5 représente ce même maillot M, toujours vu de face, porté par l'utilisateur U ;
- la figure 6 représente le même maillot M, dans la même conformation, vu en coupe VI-VI de la figure 5 ;
- la figure 7 représente, selon la section transversale VII-VII montrée à la Figure 2, l'élément longitudinal postérieur central d'ancrage transversal obtenu ou construit sur ou dans le maillot M, selon deux variantes représentées respectivement aux figures 7a et 7b.

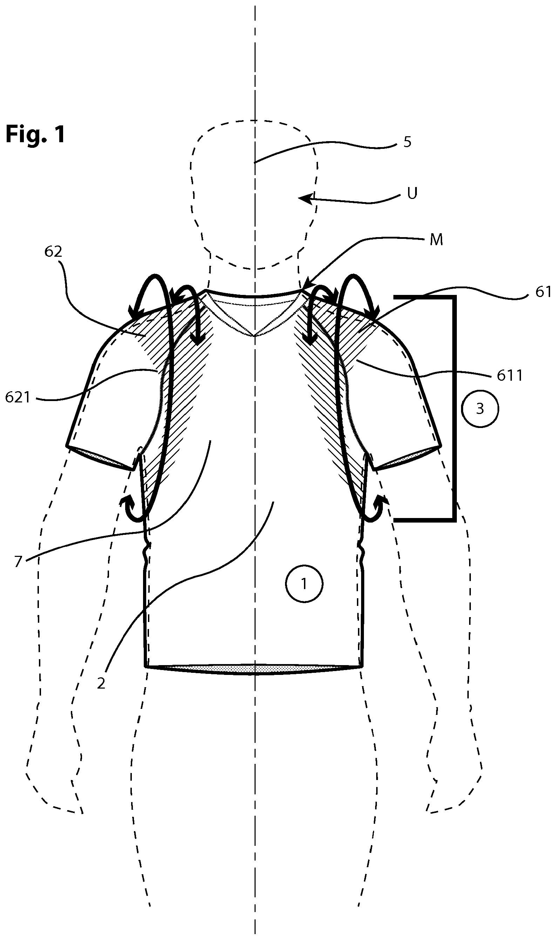

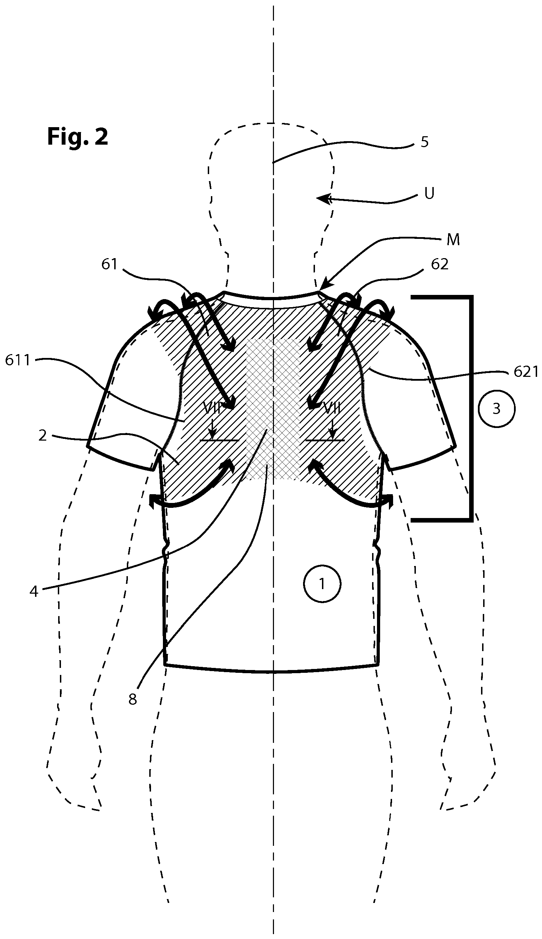

Conformément aux figures 1 à 7, l'article vestimentaire M d'assistance biomécanique au tronc 1 de l'utilisateur U est destiné à être porté ou revêtir le corps de ce dernier. Cet article M comprend ou incorpore une gaine élastique 2 d'assistance fonctionnelle à au moins un segment longitudinal d'intérêt du tronc de l'utilisateur U, par exemple son torse comprenant sa ceinture occipitale et/ou sa ceinture scapulaire, et par exemple au moins les trois vertèbres consécutives qui leur correspondent.

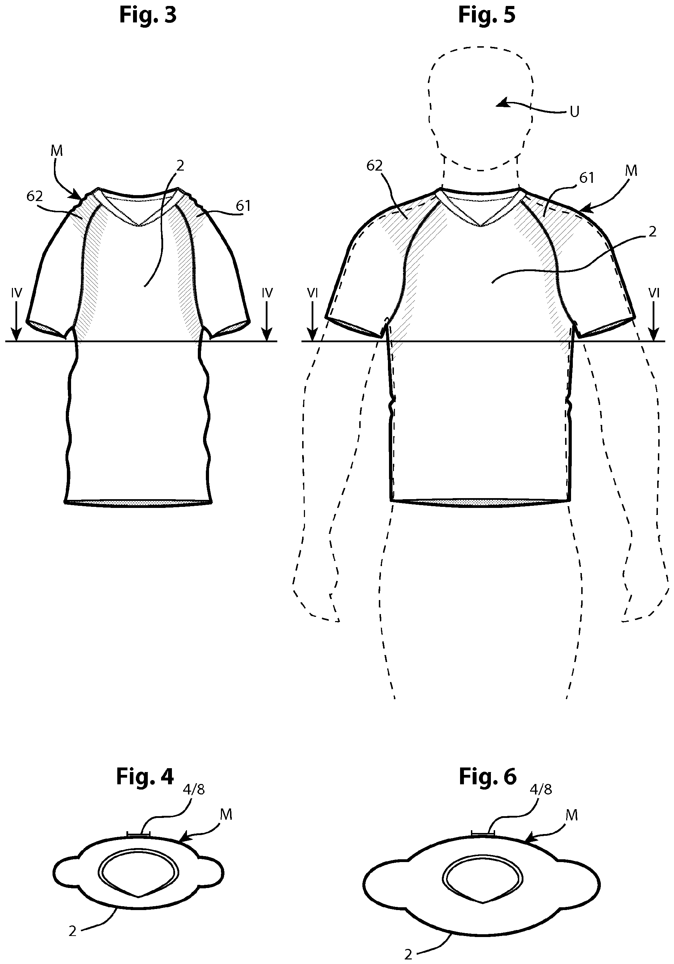

Cette gaine élastique a par construction textile, en combinaison, les caractéristiques techniques suivantes :
a) de part et d'autre de l'élément longitudinal 4 postérieur central d'ancrage transversal, défini ci-après, la gaine élastique 2 présente une élasticité périmétrique, autour de l'axe longitudinal 5 de l'article vestimentaire, et selon au moins une partie de sa hauteur, déterminant un volume interne, à l'état déployé initial (cf. Figures 3 et 4), c'est-à-dire lorsque l'article vestimentaire n'est pas porté par l'utilisateur U, au plus égal à 90 % du volume externe du segment longitudinal d'intérêt 3 du tronc de l'utilisateur U (cf. Figures 5 et 6) ;
b) cette gaine élastique 2 comporte au moins un élément longitudinal 4 postérieur central d'ancrage transversal, dont l'élasticité transversale est plus faible que son élasticité longitudinale ; cet élément longitudinal postérieur central s'alignant avec ou sur la colonne vertébrale de l'utilisateur U ayant revêtu l'article vestimentaire M, en recouvrant par exemple au moins trois vertèbres consécutives ; cette élasticité transversale de l'élément longitudinal est par exemple quasiment nulle ;
c) de part et d'autre de l'élément longitudinal 4 postérieur central d'ancrage transversal, la gaine élastique comporte deux sections textiles 61 et 62 de rappel élastique du tronc, et notamment du torse de l'utilisateur U revêtu par l'article vestimentaire M ; chaque dite section textile s'étend à ou selon la surface de la gaine 2, à partir dudit élément longitudinal 4 postérieur, vers la partie antérieure de la gaine 2, jusqu'à une bordure continue 611 ou 621, dont une ou plusieurs portions sont situées en dehors ou au-delà du côté postérieur de la gaine élastique, par exemple du côté antérieur (cf. Figure 1) ; chaque section textile 61 ou 62 de rappel élastique présente une élasticité transversale, moins importante que l'élasticité périmétrique ou transversale de la partie restante de la gaine élastique 2 ; et ces deux sections textiles de rappel élastique 61 et 62 laissant au moins une portion centrale et antérieure 7 de ladite gaine 2, libre de tout ceinturage, outre celui élastique inhérent au port de l'article vestimentaire M, et donc de la gaine élastique 2 d'assistance fonctionnelle.

Un article vestimentaire selon la présente invention est réalisé par exemple selon toutes techniques traditionnelles dans l'industrie textile, notamment l'industrie de la maille, et de la confection :
- d'abord par le choix, la découpe ou l'obtention, puis l'assemblage des pièces ou parties constitutives de l'article, par exemple au moyen d'un ou plusieurs patrons selon les tailles standardisées (par exemple L, XL, XXL selon la normalisation française ou européenne) ;
- ensuite, par le choix des constructions textiles retenues pour les différentes pièces respectivement, par exemple tricotage ou maille selon un mode préféré, mais non exclusif, de l'invention, ou tissage, ou encore non tissé ;
- et finalement, par le choix des fils retenus pour la construction textile des différentes pièces ; un fil intégrant un matériau élastomère, tel que d'élasthanne, étant préféré pour l'obtention des parties élastiques de l'article vestimentaire ; mais les matériaux textiles tels que coton, polyamide, polyester, peuvent être mis en oeuvre selon l'invention.

L'assemblage des différentes pièces ou parties constitutives de l'article vestimentaire s'effectue selon toutes techniques traditionnelles, par couture, tricotage, collage, ou broderie, par exemple.

Le mode de construction textile, par exemple de tricotage, retenu pour les différentes pièces ou parties constitutives détermine aussi leurs différentes propriétés intrinsèques, telles qu'élasticité longitudinale, transversale, ou périmétrique, résistance ou ténacité, perméabilité, "respiration", etc.

Dans certains cas, et selon l'équipement ou le matériel textile mis en oeuvre, pour construire les tissus ou étoffes requis pour l'article vestimentaire selon l'invention, par exemple avec un métier à tricoter circulaire, cet article peut être obtenu directement par une seule et même opération de tricotage ou tissage.

Un article vestimentaire selon l'invention peut présenter, à l'intérieur ou à l'extérieur, ou en dehors de la gaine élastique 2 d'assistance fonctionnelle, d'autres parties, constituants ou accessoires, ou fonctionnalités apportant d'autres propriétés ou caractéristiques à l'article, telles que :
- anti-transpiration,
- imperméabilité vis-à-vis de l'eau extérieure,
- renfort mécanique, et anti-déchirement ;
- décoration et "design" selon les tendances de la mode,
- capteurs ou éléments électroniques
- etc.

Un article vestimentaire selon l'invention peut comporter, en particulier, un système ou moyen d'ouverture/fermeture, du type fermeture à glissière, permettant à l'utilisateur de le revêtir sans avoir à l'enfiler par la tête, par exemple dans le cas d'un maillot.

Un article vestimentaire selon la présente invention fait l'objet de différents modes d'exécution.

Concernant l'élément longitudinal 4 postérieur central d'ancrage transversal, selon une variante d'exécution, celui-ci n'a pratiquement aucune élasticité transversale, selon toute sa longueur dans la direction longitudinale.

Selon une autre variante d'exécution, ce même élément longitudinal a une élasticité longitudinale relativement faible, tout en demeurant supérieure à l'élasticité transversale du même élément.

Selon une autre variante d'exécution, au plan pratique, ce même élément longitudinal 4, appartenant à la gaine élastique 2 d'assistance fonctionnelle, a la forme d'une bande 8, ou intégrée ou obtenue dans l'épaisseur de la gaine élastique 2, ou rapportée ou fixée, à l'intérieur et/ou à l'extérieur, sur cette gaine élastique 2.

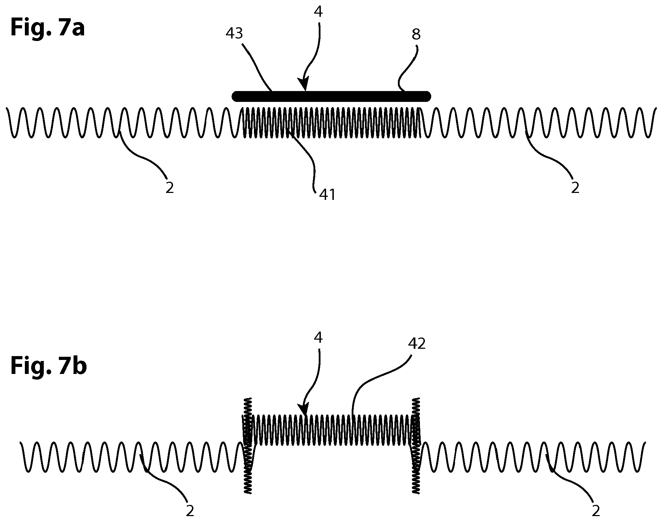

Selon la première modalité, à savoir bande 8 intégrée ou rapportée dans l'épaisseur de la gaine élastique 2, et s'agissant par exemple d'une gaine obtenue par tricotage ou tissage d'un fil élastique (à base par exemple d'élasthanne), ou non, l'élément longitudinal 4 est obtenu par une zone longitudinale 41 dont la construction textile est densifiée localement, par exemple par une maille de tricotage beaucoup plus serrée (cf. Figure 7a) à l'endroit de l'élément longitudinal 4 postérieur central d'ancrage transversal, ou en insérant ou rapportant par couture dans la gaine 2 une bande longitudinale postérieure centrale 42 présentant une densification de sa construction textile (cf. Figure 7b).

Selon la deuxième modalité, pouvant ou non être combinée à la première modalité, à savoir bande 8 rapportée ou fixée, à l'intérieur et/ou à l'extérieur, sur la gaine élastique 2, il s'agit par exemple d'une bande 8 en matériau élastomère 43, tel que du silicone ou polyuréthanne, collée, ou thermocollée, sur au moins la face extérieure du matériau textile constitutif de la gaine élastique 2, par exemple tricot élastique, ou la zone longitudinale 41 de construction textile densifiée (cf. Figure 7a).

Concernant l'élément longitudinal 4 postérieur central d'ancrage transversal, selon une autre variante d'exécution, l'élément longitudinal 4 recouvre ou se superpose à au moins trois, en particulier au moins quatre, et par exemple au moins cinq vertèbres consécutives de la colonne vertébrale de l'utilisateur U.

Comme montré par les Figures 5 et 6, de part et d'autre de l'élément longitudinal 4 postérieur central d'ancrage transversal, la gaine élastique 2 présente une élasticité périmétrique, autour de l'axe longitudinal 5 de l'article vestimentaire M, et selon au moins une partie de sa hauteur, c'est-à-dire selon la direction longitudinale de l'article vestimentaire M. Cette élasticité périmétrique, plus ou moins importante, détermine tout d'abord, lorsque l'article vestimentaire M n'est pas porté par l'utilisateur U, à l'état initial, mais dans la conformation déployée de l'article ou de la gaine, à la manière d'une robe tombant autour de la taille, un certain volume interne. C'est ce volume interne, en général peu important, rapporté au volume externe du segment longitudinal d'intérêt du tronc de l'utilisateur, c'est-à-dire s'étendant en hauteur selon l'axe longitudinal, qui détermine le taux ou l'importance de la compression élastique exercée sur le tronc de l'utilisateur U, et en particulier sur et autour dudit segment longitudinal d'intérêt 3.

Ce volume interne initial est au plus égal à 90 % du volume externe du segment longitudinal 3 d'intérêt du tronc de l'utilisateur U. Au-delà de 90 %, la compression élastique exercée sur le tronc de l'utilisateur apparaît être insuffisante pour que l'action structurante de l'élément longitudinal 4 postérieur centrage d'ancrage transversal, en liaison avec les deux sections textiles 61 et 62 de rappel élastique s'exerce efficacement sur le tronc, et notamment le torse de l'utilisateur U, au niveau du segment longitudinal d'intérêt 3.

Préférentiellement, ce volume interne initial est au plus égal à 75 %, par exemple au plus égal à 60 % du volume externe du segment longitudinal d'intérêt 3 du tronc de l'utilisateur U (cf. Figures 5 et 6).

Concernant chaque section textile 61, 62 de rappel élastique du tronc de l'utilisateur U, revêtu par l'article vestimentaire M, selon un autre mode d'exécution de l'invention, l'élasticité longitudinale de chacune est du même ordre de grandeur que l'élasticité longitudinale dans au moins une partie, par exemple dans le reste de l'article vestimentaire, c'est-à-dire en dehors ou à l'extérieur de la gaine élastique 2.

Un article vestimentaire selon la présente invention peut avoir différentes formes, selon son usage ou sa destination. Il peut s'agir d'un vêtement complet habillant complètement le tronc de l'utilisateur, intégrant ou comprenant, de manière apparente ou non, à titre de partie ou composant, la gaine d'assistance fonctionnelle définie ou décrite précédemment. Un tel vêtement sera porté par l'utilisateur de manière permanente, ou occasionnelle, selon l'assistance biomécanique dont il a besoin. Et ce vêtement fera partie de la garde-robe de l'utilisateur, au même titre que ses autres vêtements.

Il peut aussi s'agir d'un sous-vêtement, tel un maillot ou une brassière. Il peut aussi s'agir d'un survêtement, auquel cas l'article vestimentaire selon l'invention est porté sur ou au-dessus d'un vêtement traditionnel ou habituel de son tronc, tel un corsage ou une chemise.

Au plan pratique, cet article vestimentaire peut recevoir différents qualitatifs ou appellations rattachés à la dénomination usuelle des vêtements, tels que maillot ou T-shirt, brassière, combinaison, combishort, haut technique, veste, débardeur, etc....

Un article vestimentaire d'assistance biomécanique selon la présente invention a différentes applications, ou différents usages, au rang desquels on peut citer :
- la pratique d'un sport, ce vêtement contribuant, lorsqu'il est porté par le sportif, principalement au rappel en position naturelle de son tronc, notamment de son torse, mais aussi à la contention physique et/ou compression de tout ou partie de son tronc ;
- la pratique d'un métier ou activité professionnelle dans laquelle le tronc, et notamment le torse du professionnel se trouve engagé ou mis physiquement à l'épreuve ;
- le traitement orthopédique ou physio-thérapeutique du tronc, et notamment du torse, en ce compris la colonne vertébrale, le bassin et les épaules, ainsi qu'à titre de vêtement orthétique, pour protéger, immobiliser ou soutenir tout ou partie du tronc de l'utilisateur portant le vêtement ;
- le confort ou bien être de l'utilisateur au niveau du tronc, en ce compris la stimulation (contraction et/ou inhibition) de ses muscles, notamment dorsaux, le soulagement des muscles ou la réduction des tensions musculaires, en particulier dorsales, le maintien ou rappel à l'équilibre de ses différents segments, la correction ou le soutien de la posture de l'utilisateur, l'alignement de ses différentes ceintures (occipitale, scapulaire, sacro-iliaque) en favorisant l'alignement du tronc, et l'ouverture facilitée ou accompagnée de la cage thoracique, favorisant une meilleure respiration et donc oxygénation de l'utilisateur.

## Revendications

1. Article vestimentaire (M) d'assistance biomécanique au torse de l'utilisateur (U) ayant revêtu ledit article, ce dernier comprenant ou incorporant une gaine élastique (2) d'assistance fonctionnelle à au moins un segment longitudinal d'intérêt (3) du tronc de l'utilisateur ; cette gaine élastique (2) ayant, par construction textile, en combinaison, les caractéristiques techniques suivantes :
a) de part et d'autre de l'élément longitudinal (4) postérieur, défini ci-après, la gaine élastique (2) présente une élasticité périmétrique, autour de l'axe longitudinal (5) de l'article vestimentaire, et selon au moins une partie de sa hauteur;
b) cette gaine élastique (2) comporte au moins un élément longitudinal (4) postérieur central, s'alignant avec ou sur la colonne vertébrale de l'utilisateur (U) ayant revêtu l'article vestimentaire (M) ;
c) de part et d'autre de l'élément longitudinal (4) postérieur central, la gaine élastique comporte deux rappels (61, 62) du tronc de l'utilisateur (U) revêtu par l'article vestimentaire (M) ; chaque dit rappel s'étend à ou selon la surface de la gaine (2), à partir dudit élément longitudinal (4) postérieur central, vers la partie antérieure de la gaine (2) ; et ces deux rappels (61, 62) laissant au moins une portion centrale et antérieure (7) de ladite gaine (2), libre de tout ceinturage, outre celui élastique inhérent au port de l'article vestimentaire (M), et donc de la gaine élastique (2) d'assistance fonctionnelle ; et
d) l'élasticité périmétrique de la gaine élastique (2) détermine un volume interne, à l'état déployé, initial, c'est-à-dire lorsque l'article vestimentaire (M) n'est pas porté par l'utilisateur (U), au plus égal à 90 % du volume externe du segment longitudinal d'intérêt (3) du tronc de l'utilisateur (U) ;
e) chaque rappel (61 ou 62) du tronc de l'utilisateur (U) comporte une section textile s'étendant jusqu'à une bordure continue (611 ou 621) comprenant une ou plusieurs portions situées en dehors ou au-delà du côté postérieur de la gaine élastique (2), notamment du côté antérieur à cette dernière, et présentant une élasticité transversale, moins importante que l'élasticité périmétrique ou transversale de la gaine élastique (2),
**caractérisé en ce que** :
f) l'élément longitudinal (4) postérieur central est un élément d'ancrage transversal de la gaine élastique (2), ayant une élasticité transversale plus faible que son élasticité longitudinale.

2. Article selon la revendication 1, **caractérisé en ce que** l'élément longitudinal (4) postérieur central d'ancrage transversal n'a pratiquement aucune élasticité transversale, selon toute sa longueur dans la direction longitudinale.

3. Article selon la revendication 1 ou 2, **caractérisé en ce que** l'élément longitudinal (4) postérieur central d'ancrage transversal a la forme d'une bande (8) intégrée ou comprise dans l'épaisseur de la gaine élastique (2) d'assistance fonctionnelle, ou fixée, à l'intérieur et/ou à l'extérieur, de cette dernière.

4. Article selon l'une quelconque des revendications 1 à 3, **caractérisé en ce que**, porté par l'utilisateur (U), l'élément longitudinal (4) postérieur central d'ancrage transversal recouvre au moins trois, en particulier au mois quatre, et par exemple au moins cinq vertèbres consécutives de la colonne vertébrale de l'utilisateur (U).

5. Article selon l'une quelconque des revendications 1 à 4, **caractérisé en ce que**, du fait de la construction textile de la gaine élastique (2) d'assistance fonctionnelle, le volume interne, à l'état déployé, initial, c'est-à-dire lorsque l'article vestimentaire (M) n'est pas porté par l'utilisateur (U), est au plus égal à 75 %, par exemple au plus égal à 60 % du volume externe du segment longitudinal d'intérêt (3) du tronc de l'utilisateur (U).

6. Article selon l'une quelconque des revendications 1 à 5, **caractérisé en ce que** chaque section textile (61, 62) de rappel élastique du tronc de l'utilisateur (U), revêtu par l'article vestimentaire (M), présente une élasticité longitudinale du même ordre de grandeur que l'élasticité longitudinale dans au moins une partie du même article, distincte de la gaine élastique (2).

## Patentansprüche

1. Bekleidungsstück (M) zur biomechanischen Unterstützung des Oberkörpers des Benutzers (U), der mit diesem Bekleidungsstück bekleidet ist, wobei letzteres eine elastische Hülle (2) zur funktionellen Unterstützung mindestens eines interessierenden Längsabschnitts (3) des Oberkörpers des Benutzers umfasst oder integriert; wobei diese elastische Hülle (2) aufgrund ihrer textilen Konstruktion die folgenden technischen Merkmale, in Kombination, aufweist:
a) beiderseits des nachstehend definierten rückseitigen Längselements (4) weist die elastische Hülle (2) eine Umfangselastizität um die Längsachse (5) des Bekleidungsstücks herum und auf mindestens einem Teil seiner Höhe auf;
b) diese elastische Hülle (2) umfasst mindestens ein rückseitiges zentrales Längselement (4), das mit der Wirbelsäule des mit dem Bekleidungsstück (M) bekleideten Benutzers ausgerichtet ist;
c) beiderseits des rückseitigen zentralen Längselements (4) umfasst die elastische Hülle zwei Rücksteller (61, 62) für den Oberkörper des mit dem Bekleidungsstück (M) bekleideten Benutzers (U); jeder dieser Rücksteller erstreckt sich auf oder entlang der Oberfläche der Hülle (2) vom rückseitigen zentralen Längselement (4) zum vorderen Teil der Hülle (2); und wobei diese zwei Rücksteller (61, 62) mindestens einen zentralen und vorderen Abschnitt (7) der Hülle (2) frei von jeder Umgürtung lassen, bis auf der elastischen, die auf das Tragen des Kleidungsstücks (M) und somit auf die elastische Hülle (2) zur funktionellen Unterstützung zurückzuführen ist; und
d) die Umfangselastizität der elastischen Hülle (2) im ausgebreiteten Anfangszustand, d.h., wenn das Bekleidungsstück (M) nicht vom Benutzer (U) getragen wird, ein Innenvolumen bestimmt, das höchstens 90 % des Außenvolumens des interessierenden Längssegments (3) des Oberkörpers des Benutzers (U) entspricht;
e) jeder Rücksteller (61 oder 62) für den Oberkörper des Benutzers (U) einen Textilabschnitt umfasst, der sich bis zu einem durchgehenden Rand (611 oder 621) erstreckt, der einen oder mehrere Abschnitte umfasst, die außerhalb oder jenseits der Rückseite der elastischen Hülle (2), insbesondere auf deren Vorderseite liegen und eine Querelastizität aufweisen, die geringer ist als die Umfangs- oder Querelastizität der elastischen Hülle (2),
**dadurch gekennzeichnet, dass**:
f) das rückseitige zentrale Längselement (4) ein Element zur Querverankerung der elastischen Hülle (2) ist, dessen Querelastizität geringer als seine Längselastizität ist.

2. Bekleidungsstück nach Anspruch 1, **dadurch gekennzeichnet, dass** das rückseitige zentrale Längselement (4) zur Querverankerung auf seiner gesamten Länge in der Längsrichtung praktisch keine Querelastizität aufweist.

3. Bekleidungsstück nach Anspruch 1 oder 2, **dadurch gekennzeichnet, dass** das rückseitige zentrale Längselement (4) zur Querverankerung die Form eines Streifens (8) aufweist, der in die Dicke der elastischen Hülle (2) zur funktionellen Unterstützung integriert oder darin enthalten ist oder an deren Innenseite und/oder Außenseite befestigt ist.

4. Bekleidungsstück nach einem der Ansprüche 1 bis 3, **dadurch gekennzeichnet, dass** das rückseitige zentrale Längselement (4) zur Querverankerung, wenn es vom Benutzer (U) getragen wird, mindestens drei, insbesondere mindestens vier und zum Beispiel mindestens fünf aufeinanderfolgende Wirbel der Wirbelsäule des Benutzers (U) überdeckt.

5. Bekleidungsstück nach einem der Ansprüche 1 bis 4, **dadurch gekennzeichnet, dass** aufgrund der textilen Konstruktion der elastischen Hülle (2) zur funktionellen Unterstützung das Innenvolumen im ausgebreiteten Anfangszustand, d. h. wenn das Bekleidungsstück (M) nicht vom Benutzer (U) getragen wird, höchstens 75 %, zum Beispiel höchstens 60 % des Außenvolumens des interessierenden Längssegments (3) des Oberkörpers des Benutzers (U) entspricht.

6. Bekleidungsstück nach einem der Ansprüche 1 bis 5, **dadurch gekennzeichnet, dass** jeder Textilabschnitt (61, 62) zur elastischen Rückstellung des Oberkörpers des Benutzers (U), der mit dem Bekleidungsstück (M) bekleidet ist, eine Längselastizität in der gleichen Größenordnung aufweist wie die Längselastizität in mindestens einem Teil desselben Bekleidungsstücks, der von der elastischen Hülle (2) getrennt ist.

## Claims

1. Article of clothing (M) for biomechanical assistance to the torso of the user (U) having worn said article, the latter comprising or incorporating an elastic sheath (2) for functional assistance to at least one longitudinal segment of interest (3) of the user's trunk; this elastic sheath (2) having, by textile construction, in combination, the following technical characteristics:
a) on either side of the posterior longitudinal element (4), defined below, the elastic sheath (2) has perimetric elasticity, around the longitudinal axis (5) of the article of clothing, and along at least a part of its height;
b) this elastic sheath (2) comprises at least one central posterior longitudinal element (4), aligned with or on the spine of the user (U) wearing the article of clothing (M);
c) on either side of the central posterior longitudinal element (4), the elastic sheath comprises two returns (61, 62) of the user's trunk (U) covered by the article of clothing (M); each said return extends at or along the surface of the sheath (2), from said central posterior longitudinal element (4), towards the anterior part of the sheath (2); and these two returns (61, 62) leaving at least one central and anterior portion (7) of said sheath (2), free from any strapping, other than the elastic strapping inherent in the wearing of the article of clothing (M), and therefore of the functional assistance elastic sheath (2); and
d) the perimeter elasticity of the elastic sheath (2) determines an internal volume, in the initial, deployed state, i.e. when the article of clothing (M) is not worn by the user (U), at most equal to 90% of the external volume of the longitudinal segment of interest (3) of the user's trunk (U);
e) each return (61 or 62) of the user's trunk (U) comprises a textile section extending to a continuous edge (611 or 621) comprising one or more portions located outside or beyond the posterior side of the elastic sheath (2), in particular the side anterior to the latter, and having a transverse elasticity, less important than the perimetric or transverse elasticity of the elastic sheath (2),
**characterized in that**:
f) the central posterior longitudinal element (4) is a transverse anchoring element for the elastic sheath (2), having a lower transverse elasticity than its longitudinal elasticity.

2. Article according to claim 1, **characterized in that** the central posterior longitudinal element (4) for transverse anchoring has virtually no transverse elasticity along its entire length in the longitudinal direction.

3. Article according to claim 1 or 2, **characterized in that** the central posterior longitudinal element (4) for transverse anchoring is in the form of a strip (8) integrated into or included in the thickness of the functional assistance elastic sheath (2), or fixed to the inside and/or outside thereof.

4. Article according to any one of claims 1 to 3, **characterized in that**, worn by the user (U), the central posterior longitudinal element (4) for transverse anchoring covers at least three, in particular at least four, and for example at least five consecutive vertebrae of the user's (U) spinal column.

5. Article according to any one of claims 1 to 4, **characterized in that**, due to the textile construction of the functional assistance elastic sheath (2), the internal volume, in the initial, deployed state, i.e. when the article of clothing (M) is not worn by the user (U), is at most 75%, for example at most 60%, of the external volume of the longitudinal segment of interest (3) of the user's trunk (U).

6. Article according to any one of claims 1 to 5, **characterized in that** each textile section (61, 62) for elastic return of the user's trunk (U), covered by the article of clothing (M), has a longitudinal elasticity of the same order of magnitude as the longitudinal elasticity in at least one part of the same article, distinct from the elastic sheath (2).
